# EUROPÄISCHE PATENTSCHRIFT

(11) **EP 0 475 353 B1**
(45) Veröffentlichungstag und Bekanntmachung des Hinweises auf die Patenterteilung: **20.12.1995**
(21) Anmeldenummer: 91115281.7
(22) Anmeldetag: 10.09.1991
(51) Int. Cl.: C07C 17/42, C09K 5/00, C07B 63/04

(54) **Stabilisierung von CF3-CHCl2 oder CClF2-CHClF oder deren Mischungen gegen die Reaktion mit Kältemaschinenöl**
Stabilisation of CF3-CHCl2 or CClF2-CHClF or their mixture against reactions with refrigerant oils
Stabilisation de CF3-CHCl2 ou CClF2-CHClF ou de leur mélange vis-à-vis des réactions avec les huiles de réfrigérant

(30) Priorität: 11.09.1990 DE 4028747
(43) Veröffentlichungstag der Anmeldung: 18.03.1992
(73) Patentinhaber: HOECHST AKTIENGESELLSCHAFT, 65926 Frankfurt am Main (DE)
(72) Erfinder: Hopp, Peter, Dr., W-6238 Hofheim am Taunus (DE); Behme, Klaus-Jürgen, W-6239 Eppstein/Taunus (DE); Deger, Hans-Matthias, Dr., W-6238 Hofheim am Taunus (DE)

(56) Entgegenhaltungen:
- EP-A- 0 062 516
- EP-A- 0 076 471
- EP-A- 0 136 683
- US-A- 3 627 834
- CHEMICAL ABSTRACTS, Band 112, Nr. 19, 7. Mai 1990, Seite 680, Zusammenfassung Nr. 178060p, Columbus, Ohio, US
- PATENT ABSTRACTS OF JAPAN, Band 9, Nr.254 (C-308)(1977), 11.Oktober 1985

## Beschreibung

Die Erfindung betrifft die Stabilisierung der noch ein unsubstituiertes Wasserstoffatom enthaltenden Fluorchlorkohlenwasserstoffe CF₃-CHCl₂ (R123) oder CClF₂-CHClF (R123a) oder deren Mischungen gegen die Reaktion mit Kältemaschinenöl. Kältemaschinenöle sind Schmierstoffe, die wegen ihrer niedrigen Viskosität und ihrer Mischbarkeit mit Kältemitteln für den Einsatz in Kühlgeräten geeignet sind. Dabei handelt es sich um Mineralöle, vorzugsweise solche auf "Naphthenbasis", d.h. naphthenreiche Mineralöle, oder um synthetische Alkylaromaten. Bevorzugt für mit R123 bzw. R123a betriebene Anlagen sind synthetische Alkylaromaten, z.B. die unter dem Namen ®Zerol 150 im Handel erhältliche Verbindung 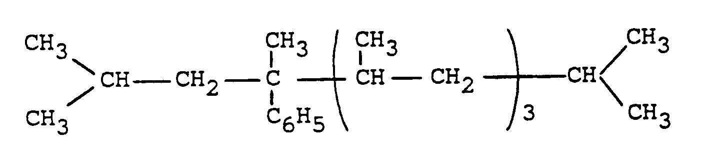
R123 und R123a, insbesondere R123, sind als Ersatzstoffe für das bisher als Kältemittel in sogenannten Kaltwassersätzen benutzte CCl₃F (R11) vorgesehen. Derartige Kältemittel müssen stabil gegen Alterungsprozesse und gegenüber Werkstoffen der Kältemaschinen und dem für die Schmierung der bewegten Teile der Kältemaschinen notwendigen Kältemaschinenöl sein.

Es hat sich jedoch gezeigt, daß Kältemaschinenöle mit R123 und R123a reagieren, insbesondere in Gegenwart üblicher Konstruktionswerkstoffe (Fe, Cu, Al) für Kältemaschinen. Als Zerfallsprodukte kann man Chlorwasserstoff und R133a (CF₃-CH₂Cl) nachweisen, und das Kältemaschinenöl, insbesondere solches auf Mineralölbasis, verfärbt sich und wird zunehmend viskoser. Der Chlorwasserstoff schädigt die Konstruktionswerkstoffe durch Korrosion, R133a ist als carcinogen verdächtig, und das Kältemaschinenöl verschlechtert seine Schmiereigenschaften.

Aus EP-A-62516 ist die Stabilisierung von Fluorkohlenwasserstoffen in Gegenwart von Amiden oder Glykolethern mittels Phosphit bekannt.

Aus EP-A2-136 683 ist die Stabilisierung von Fluorchlorkohlenwasserstoffen gegenüber einer Reaktion mit Schmierölen, insbesondere in Gegenwart von Metallen bekannt; dabei werden Borverbindungen als Stabilisatoren eingesetzt. Weiter ist bereits bekannt, die Zersetzung von CCl₃-CF₃ (R113a) und CCl₃F (R11) in Gegenwart von Hydroxylgruppen enthaltenden Aminen durch die Zugabe von 2,4-Diphenyl-4-methylpenten (DMP) zu verhindern (DE-PS 3 139 401).

Es wurde nun überraschenderweise gefunden, daß DMP auch R123 und R123a gegen die Reaktion mit Kältemaschinenöl stabilisiert. Das war deswegen nicht zu erwarten, weil gemäß DE-PS 3 139 401 das DMP benutzt wird, um die Oxidation eines Aminoalkohols zu einem Aminoaldehyd zu unterdrücken, während es im vorliegenden Fall darum geht, einen Austausch von Chloratomen gegen Wasserstoffatome zu verhindern.

Ein Gegenstand der Erfindung sind Mischungen, gemäß Anspruch 1.

Ein weiterer Gegenstand der Erfindung ist ein Verfahren gemäß Anspruch 4.

Die Menge an zugesetztem DMP ist so zu bemessen, daß eine ausreichende Stabilisierung erreicht wird; eine wesentlich größere als die hierfür notwendige Menge zuzusetzen, ist nicht zu empfehlen, sowohl aus Kostengründen als auch weil dadurch der Anteil an Fluorchlorkohlenwasserstoff in der Mischung aus Fluorchlorkohlenwasserstoff und DMP zu stark herabgesetzt werden könnte, so daß die thermodynamischen und thermophysikalischen Eigenschaften dieser Mischung beeinträchtigt würden. Im allgemeinen setzt man 0,01 bis 10 Gew.-% DMP zu, vorzugsweise 0,2 bis 2 Gew.-% DMP, insbesondere 0,5 bis 1 Gew.-% DMP, wobei die Gewichtsprozente stets auf das Gewicht des oder der eingesetzten Fluorchlorkohlenwasserstoffe bezogen sind. Bei Temperaturen unter 130°C wird die Dechlorierung der Fluorchlorkohlenwasserstoffe durch das DMP vollständig unterdrückt, und selbst bei Temperaturen von 150°C und mehr, die lediglich zum Testen angewandt werden, um jahrelange Dauertests bei niedrigeren Temperaturen zu simulieren, wird die Dechlorierung noch stark vermindert.

Das 2,4-Diphenyl-4-methyl-penten besteht im allgemeinen aus den beiden Isomeren 2,4-Diphenyl-4-methyl-penten-(1) und 2,4-Diphenyl-4-methyl-penten-(2). Das Isomerenverhältnis Penten-(1)/Penten-(2) beträgt dabei im allgemeinen etwa 1:1 bis 9:1.

Anstelle des Gemisches der beiden Pentene läßt sich auch ein technisches Gemisch einsetzen, das neben diesen beiden Isomeren noch einen geringen Anteil an 1,1,3-Trimethyl-3-phenylindan enthält. Ein solches Gemisch ist z.B. nach dem Verfahren der US-PS 2 429 719 erhältlich und enthält im allgemeinen 45 bis 89 Gew.-% 2,4-Diphenyl-4-methyl-penten-(1), 10 bis 45 Gew.-% 2,4-Diphenyl-4-methyl-penten-(2) und 1 bis 20 Gew.-% 1,1,3-Trimethyl-3-phenylindan. Bei Einsatz eines solchen technischen Gemischs muß natürlich eine solche Menge gewählt werden, daß die oben angegebenen Prozentgehalte an reinem DMP erreicht werden.

Natürlich kann man auch die reinen Isomeren einzeln einsetzen, aber eine Trennung des bei der Herstellung des DMP entstehenden Isomerengemisches wäre ein unnötiger Aufwand.

DMP ist nicht selbstentzündlich, nicht explosionsgefährlich, nicht luftempfindlich und neigt nicht zur Polymerisation.

Der Flammpunkt liegt bei 120 °C, so daß DMP nicht mehr zur Gefahrenklasse der brennbaren Flüssigkeiten gehört. Es ist mit dem oder den Fluorchlorkohlenwasserstoffen und dem Kältemaschinenöl in jedem Verhältnis bis zur tiefsten üblicherweise infrage kommenden Verdampfungstemperatur von -2 °C und der normalerweise in solchen Kältemaschinen höchstens erreichten Verflüssigungstemperatur von ca. 50°C mischbar.

### Beispiele

Es wurden jeweils ®Zerol 150 als Kältemaschinenöl, R123 und DMP mit insgesamt 2 g Metallsplittern (Aluminium, Kupfer und Eisen) in ein Bombenrohr eingefüllt und bei 150 °C getempert. In den Vergleichsbeispielen 1, 2, 3 wurde auf den Stabilisator DMP verzichtet, bei sonst gleichem Vorgehen. Die Bildung von R133a wurde gaschromatographisch nach Ablauf der in den Beispielen angegebenen Zeiten t bestimmt. Die angegebenen Prozentzahlen sind Gewichtsprozente.

### Beispiel 1

Zusammensetzung der Mischung:
3,0 g (57,5 %) R123
1,7 g (32,5 %) ®Zerol 150
0,5 g (9,5 %) DMP
Versuchsdauer t = 70 h
Gefunden: 125 ppm R133a (bezogen auf R123).

### Vergleichsbeispiel 1

Versuchsdauer und Zusammensetzung wie in Beispiel 1, jedoch ohne DMP.
Gefunden: 1300 ppm R133a (bezogen auf R123).

### Beispiel 2

Zusammensetzung der Mischung:
3,0 g (92,5 %) R123
0,2 g (6,0 %) ®Zerol 150
0,05 g (1,5 %) DMP
Versuchdauer t = 64 h
R133a nicht nachweisbar.

### Vergleichsbeispiel 2

Versuchsdauer und Zusammensetzung wie in Beispiel 2, jedoch ohne DMP.
Gefunden: 10 ppm R133a (bezogen auf R123).

### Beispiel 3

Zusammensetzung der Mischung:
3,0 g (85,5 %) R123
0,5 g (14,0 %) ®Zerol 150
0,02 g (0,5 %) DMP
Versuchsdauer t = 91 h
R133a nicht nachweisbar.

### Vergleichsbeispiel 3

Versuchsdauer und Zusammensetzung wie in Beispiel 3, jedoch ohne DMP.
Gefunden: 20 ppm R133a (bezogen auf R123).

## Patentansprüche (Patentansprüche für folgende(n) Vertragsstaat(en): BE, DE, DK, FR, GB, GR, IT, LU, NL)

1. Mischung, die im wesentlichen aus 2,4-Diphenyl-4-methyl-penten-(1) und/oder -penten-(2) und mindestens einer der Verbindungen CF₃-CHCl₂ und CClF₂-CHClF besteht und die 0,01 bis 10 Gew.-% 2,4-Diphenyl-4-methyl-penten-(1) und/oder -penten-(2) enthält, bezogen auf die Menge an Fluorchlorkohlenwasserstoff.

2. Mischung nach Anspruch 1, die 0,2 bis 2 Gew.-% 2,4-Diphenyl-4-methyl-penten-(1) und/oder -penten-(2) enthält, bezogen auf die Menge an Fluorchlorkohlenwasserstoff.

3. Mischung nach Anspruch 1, die 0,5 bis 1 Gew.-% 2,4-Diphenyl-4-methyl-penten-(1) und/oder -penten-(2) enthält, bezogen auf die Menge an Fluorchlorkohlenwasserstoff.

4. Verfahren zur Stabilisierung von CF₃-CHCl₂ oder CClF₂-CHClF oder deren Mischungen gegen die Reaktion mit Kältemaschinenöl, dadurch gekennzeichnet, daß man dem Fluorchlorkohlenwasserstoff oder dem Gemisch der beiden Fluorchlorkohlenwasserstoffe 0,01 bis 10 Gew.-% 2,4-Diphenyl-4-methyl-penten-(1) und/oder -penten-(2), bezogen auf die Menge an Fluorchlorkohlenwasserstoff zusetzt.

5. Verfahren nach Anspruch 4, dadurch gekennzeichnet, daß man 0,2 bis 2 Gew.-% 2,4-Diphenyl-4-methyl-penten-(1) und/oder -penten-(2), bezogen auf die Menge an Fluorchlorkohlenwasserstoff zusetzt.

6. Verfahren nach Anspruch 4, dadurch gekennzeichnet, daß man 0,5 bis 1 Gew.-% 2,4-Diphenyl-4-methyl-penten-(1) und/oder -penten-(2), bezogen auf die Menge an Fluorchlorkohlenwasserstoff zusetzt.

## Patentansprüche (Patentansprüche für folgende(n) Vertragsstaat(en): ES)

1. Verfahren zur Stabilisierung von CF₃-CHCl₂ oder CClF₂-CHClF oder deren Mischungen gegen die Reaktion mit Kältemaschinenöl, dadurch gekennzeichnet, daß man dem Fluorchlorkohlenwasserstoff oder dem Gemisch der beiden Fluorchlorkohlenwasserstoffe 0,01 bis 10 Gew.-% 2,4-Diphenyl-4-methyl-penten-(1) und/oder -penten-(2), bezogen auf die Menge an Fluorchlorkohlenwasserstoff zusetzt.

2. Verfahren nach Anspruch 1, dadurch gekennzeichnet, daß man 0,2 bis 2 Gew.-% 2,4-Diphenyl-4-methyl-penten-(1) und/oder -penten-(2), bezogen auf die Menge an Fluorchlorkohlenwasserstoff zusetzt.

3. Verfahren nach Anspruch 1, dadurch gekennzeichnet, daß man 0,5 bis 1 Gew.-% 2,4-Diphenyl-4-methyl-penten-(1) und/oder -penten-(2), bezogen auf die Menge an Fluorchlorkohlenwasserstoff zusetzt.

## Claims (Claims for the following Contracting State(s): BE, DE, DK, FR, GB, GR, IT, LU, NL)

1. A mixture which essentially comprises 2,4-diphenyl-4-methylpent-1-ene and/or -pent-2-ene and at least one of the compounds CF₃-CHCl₂ and CClF₂-CHClF and which contains 0.01 to 10 % by weight of 2,4-diphenyl-4-methylpent-1-ene and/or -pent-2-ene, based on the amount of chlorofluorohydrocarbon.

2. A mixture as claimed in claim 1, which contains 0.2 to 2 % by weight of 2,4-diphenyl-4-methylpent-1-ene and/or -pent-2-ene, based on the amount of chlorofluorohydrocarbon.

3. A mixture as claimed in claim 1, which contains 0.5 to 1 % by weight of 2,4-diphenyl-4-methylpent-1-ene and/or -pent-2-ene, based on the amount of chlorofluorohydrocarbon.

4. A process for the stabilization of CF₃-CHCl₂ or CClF₂-CHClF or a mixture thereof against reaction with refrigerating machinery oil, which comprises adding 0.01 to 10 % by weight of 2,4-diphenyl-4-methylpent-1-ene and/or -pent-2-ene, based on the amount of chlorofluorohydrocarbon, to the chlorofluorohydrocarbon or the mixture of the two chlorofluorohydrocarbons.

5. The process as claimed in claim 4, wherein 0.2 to 2 % by weight of 2,4-diphenyl-4-methylpent-1-ene and/or -pent-2-ene is added, based on the amount of chlorofluorohydrocarbon.

6. The process as claimed in claim 4, wherein 0.5 to 1 % by weight of 2,4-diphenyl-4-methylpent-1-ene and/or -pent-2-ene is added, based on the amount of chlorofluorohydrocarbon.

## Claims (Claims for the following Contracting State(s): ES)

1. A process for the stabilization of CF₃-CHCl₂ or CClF₂-CHClF or a mixture thereof against reaction with refrigerating machinery oil, which comprises adding 0.01 to 10 % by weight of 2,4-diphenyl-4-methylpent-1-ene and/or -pent-2-ene, based on the amount of chlorofluorohydrocarbon, to the chlorofluorohydrocarbon or the mixture of the two chlorofluorohydrocarbons.

2. The process as claimed in claim 1, wherein 0.2 to 2 % by weight of 2,4-diphenyl-4-methylpent-1-ene and/or -pent-2-ene is added, based on the amount of chlorofluorohydrocarbon.

3. The process as claimed in claim 1, wherein 0.5 to 1 % by weight of 2,4-diphenyl-4-methylpent-1-ene and/or -pent-2-ene is added, based on the amount of chlorofluorohydrocarbon.

## Revendications (Revendications pour l'(les) Etat(s) contractant(s) suivant(s): BE, DE, DK, FR, GB, GR, IT, LU, NL)

1. Mélange qui se compose essentiellement de 2,4-diphényl-4-méthylpentène-(1) et/ou -pentène-(2) et au moins d'un des composés CF₃-CHCl₂ et CClF₂-CHClF et contient de 0,01 à 10 % en poids de 2,4-diphényl-4-méthylpentène-(1) et/ou -pentène-(2) par rapport à la quantité de l'hydrocarbure fluorochloré.

2. Mélange selon la revendication 1, qui contient de 0,2 à 2 % en poids de 2,4-diphényl-4-méthylpentène-(1) et/ou -pentène-(2) par rapport à la quantité de l'hydrocarbure fluorochloré.

3. Mélange selon la revendication 1, qui contient de 0,5 à 1 % en poids de 2,4-diphényl-4-méthylpentène-(1) et/ou -pentène-(2) par rapport à la quantité de l'hydrocarbure fluorochloré.

4. Procédé pour la stabilisation de CF₃-CHCl₂ ou CClF₂-CHClF ou de leurs mélanges contre la réaction avec l'huile pour machines frigorifiques, caractérisé en ce qu'on ajoute à l'hydrocarbure fluorochloré ou au mélange des deux hydrocarbures fluorochlorés de 0,01 à 10 % en poids de 2,4-diphényl-4-méthylpentène-(1) et/ou -pentène-(2) par rapport à la quantité de l'hydrocarbure fluorochloré.

5. Procédé selon la revendication 4, caractérisé en ce qu'on ajoute 0,2 à 2 % en poids de 2,4-diphényl-4-méthylpentène-(1) et/ou -pentène-(2) calculé par rapport à la quantité de l'hydrocarbure fluorochloré.

6. Procédé selon la revendication 4, caractérisé en ce qu'on ajoute 0,5 à 1 % en poids de 2,4-diphényl-4-méthylpentène-(1) et/ou -pentène-(2) calculée par rapport à la quantité de l'hydrocarbure fluorochloré.

## Revendications (Revendications pour l'(les) Etat(s) contractant(s) suivant(s): ES)

1. Procédé de stabilisation de CF₃-CHCl₂ ou CClF₂-CHClF ou leurs mélanges contre la réaction avec l'huile pour machines frigorifiques, caractérisé en ce qu'on ajoute à l'hydrocarbure fluorochloré ou au mélange des deux hydrocarbures fluorochlorés de 0,01 à 10% en poids de 2,4-diphényl-4-méthylpentène-(1) et/ou -pentène-(2), par rapport à la quantité de l'hydrocarbure fluorochloré.

2. Procédé selon la revendication 1, caractérisé en ce qu'on ajoute de 0,2 à 2 % en poids de 2,4-diphényl-4-méthylpentène-(1) et/ou -pentène-(2) par rapport à la quantité de l'hydrocarbure fluorochloré.

3. Procédé selon la revendication 1, caractérisé en ce qu'on ajoute de 0,5 à 1 % en poids de 2,4-diphényl-4-méthylpentène-(1) et/ou -pentène-(2) par rapport à la quantité de l'hydrocarbure fluorochloré.
